# EUROPEAN PATENT SPECIFICATION

(11) **EP 4 116 323 B1**
(45) Date of publication and mention of the grant of the patent: **08.07.2026**
(21) Application number: 21788631.6
(22) Date of filing: 15.04.2021
(51) Int. Cl.: C07K 16/08, C12P 21/08, G01N 33/569, C12N 15/13

(54) **ADENOVIRUS IMMUNOASSAY METHOD AND IMMUNOASSAY INSTRUMENT**
ADENOVIRUS-IMMUNTESTVERFAHREN UND IMMUNTESTINSTRUMENT
PROCÉDÉ ET INSTRUMENT DE DOSAGE IMMUNOLOGIQUE D'ADÉNOVIRUS

(30) Priority: 16.04.2020 JP 2020073236
(43) Date of publication of application: 11.01.2023
(73) Proprietor: Denka Company Limited, Tokyo 103-8338 (JP)
(72) Inventor: MIYAZAWA, Takashi, Gosen-shi, Niigata 959-1834 (JP); KUWAHARA, Miwa, Gosen-shi, Niigata 959-1834 (JP)
(74) Representative: Mewburn Ellis LLP
(86) International application number: PCT/JP2021/015540
(87) International publication number: WO 2021/210632

(56) References cited:
- CN-A- 108 794 622
- JP-A- 2008 122 372
- QIU HONGLING, ZHOU ZHICHAO, FENG KAIXIA, TIAN XINGUI, LI XIAO, LI HAITAO, XING KE, CHEN JINGXIAN, LI CHUANJIANG, ZHOU RONG: "Epitope mapping and cross-reactivity analysis of the monoclonal antibodies against hexon protein of human adenovirus type 3", VIRUS RESEARCH, vol. 146, no. 1-2, 1 December 2009 (2009-12-01), pages 58 - 65, XP055857567, DOI: 10.1016/j.virusres.2009.08.011
- TIAN XINGUI; LI CHENYANG; XUE CHUNYAN; LI XIAO; ZHOU ZHICHAO; ZHOU RONG: "Epitope mapping and characterization of a neutralizing monoclonal antibody against human adenovirus type 3", VIRUS RESEARCH, vol. 177, no. 2, 1 November 2013 (2013-11-01), pages 189 - 193, XP028735780
- DATABASE Nucleotide 14 April 2008 (2008-04-14), ANONYMOUS: "Human adenovirus 3 gene for hexon, complete cds, strain: GB", XP055857576, retrieved from GENBANK Database accession no. AB330084
- XIAO-HUI YUAN., YING-CHEN WANG., WEN-JING JIN , BIN-BIN ZHAO , CAI-FENG CHEN , JIAN YANG , JING-FEI WANG , YING-YING GUO , JING-JU: "Structure-Based High-Throughput Epitope Analysis of Hexon Proteins in B and C Species Human Adenoviruses (HAdVs)", PLOS ONE, vol. 7, no. 3, e32938, 13 March 2012 (2012-03-13), pages 1 - 13, XP055857578
- MIZUTA K; MATSUZAKI Y; HONGO S; OHMI A; OKAMOTO M; NISHIMURA H; ITAGAKI T; KATSUSHIMA N; OSHITANI H; SUZUKI A; FURUSE Y; NODA M; K: "Stability of the seven hexon hypervariable region sequences of adenovirus types 1-6 isolated in Yamagata, Japan between 1988 and 2007", VIRUS RESEARCH, vol. 140, no. 1-2, 1 March 2009 (2009-03-01), pages 32 - 39, XP025991637, ISSN: 0168-1702, DOI: 10.1016/j.virusres.2008.10.014
- É. ÁDÁM, I. NÁSZ , A. LENGYEL: "Characterization of adenovirus hexons by their epitope composition", ARCHIVES OF VIROLOGY, vol. 141, no. 10, 1 October 1996 (1996-10-01), AT , pages 1891 - 1907, XP009531036, ISSN: 0304-8608, DOI: 10.1007/BF01718202

## Description

### TECHNICAL FIELD

The present invention relates to an immunoassay for adenovirus and an immunoassay device therefor, and to an anti-adenovirus antibody for the immunoassay and the device.

### BACKGROUND ART

Adenovirus is known as a pathogen of the following: a respiratory disease such as acute febrile pharyngitis, pharyngoconjunctivitis, acute airway inflammation, or viral pneumonia; an eye disease such as acute follicular conjunctivitis or epidemic keratoconjunctivitis; a gastrointestinal disease such as transmissible gastroenteritis; a urologic disease such as urethritis; or the like. At present, adenovirus is classified into seven species A to G, and there are more than 80 types of adenovirus. It has been reported that the types up to type 51 are serotypes, and that the types from type 52 and above are genotypes based on the determination of the entire base sequence (Non-patent Document 1). When humans are infected with adenovirus, the humans can exhibit various clinical symptoms, and do not often exhibit a specific pathological condition, and thus, it is difficult to prove adenovirus infection from a clinical symptom. In addition, adenovirus is very infectious, and preventing herd infection is considered to involve proving viral infection early.

Examples of methods developed to detect adenovirus rapidly and simply include an immunochromatography to be performed with an anti-adenovirus antibody and a method to be performed with EIA. However, in the ophthalmologic field where a specimen can be collected only in a small amount, the positive ratio is 60% or less, and there is a demand for a rapid higher-sensitivity diagnostic method or an anti-adenovirus monoclonal antibody usable for such a method.

The infectious disease surveillance of National Institute of Infectious Diseases provides the information that there are patients who have been found to have pharyngoconjunctival fever, transmissible gastroenteritis, or epidemic keratoconjunctivitis as an adenovirus-associated disease. It is known that acute respiratory disease and pharyngoconjunctival fever are caused by the adenovirus species B, C, and E, that transmissible gastroenteritis is caused by the adenovirus species A, F, and G, and that epidemic keratoconjunctivitis is caused by the adenovirus species B, D, and E (Non-Patent Document 2). The species B adenovirus type 3 and the species E adenovirus type 4 are the most common etiology of epidemic keratoconjunctivitis and pharyngoconjunctival fever, and the species D adenovirus type 8, type 19, and type 37 are also the causes of a serious outbreak of epidemic keratoconjunctivitis in some countries, particularly in East Asia and Southeast Asia. Adenovirus type 8, type 19, and type 37 are well known as the epidemiologic causes of nosocomial infection. Nosocomial infection induced by adenovirus has recently posed a notable social issue in public hygiene and an economical and ethical issue in hospitals.

Up to now, a plurality of monoclonal antibodies that react with adenovirus have been produced and reported. For example, disclosed is a method in which adenovirus is detected using a monoclonal antibody that reacts with a specific subtype of adenovirus (Patent Documents 1 and 2). Hongling *et al.* (Non-patent document ) reports the generation of three monoclonal antibodies (5D4, 7B2 and 3D10) against recombinant HAdV-3 hexon protein and the investigation of their epitopes. Only 5D4 was found to react with the native HAdV-3 virus particles. Tian, *et al.,* (Non-patent document 4) reports the generation of the monoclonal antibody MAb 1B6 using the HAdV-3 virion. Mab1B6 recognised the HAdV-4 virus particle and the HAdV-3 hexon protein but not the viral particle or the hexon protein of HAdV-7 and HAdV-4. JP 2008-122372 A (Patent Document 3) is directed to a method of filtering a sample to prevent pseudo false positives and assays using the same. The disclosure includes use of the assay to detect influenza virus.

### PRIOR ART DOCUMENTS

### [Non-patent Documents]

[Non-patent Document 1] Seto D, et al., J Virol 85: 5701-5702, 2011
[Non-patent Document 2] IASR Vol. 38, p.133-135: July 2017
[Non-patent Document 3] Hongling, et al., Virus Res 146: 58-65, 2009
[Non-patent Document 4] Tian, et al., Virus Res 177: 189-193, 2013
[Patent Document 1] JP 2000-290298 A
[Patent Document 2] JP 2000-290299 A
[Patent Document 3] JP 2008-122372 A

### SUMMARY OF THE INVENTION

### PROBLEMS TO BE SOLVED BY THE INVENTION

However, none of the monoclonal antibodies for adenovirus that are currently produced have sufficient detection sensitivity for adenovirus, and there is a demand for a monoclonal antibody that reacts with higher sensitivity. In addition, a conventional anti-adenovirus monoclonal antibody, for which the amino acid sequence of an epitope has not been identified, hence has a problem of poor reproducibility.

An object of the present invention is to provide: a monoclonal antibody that makes it possible that adenovirus contained in a test specimen is detected and measured rapidly, simply, and with high-sensitivity; and an immunoassay for adenovirus and an immunoassay device therefor, for both of which the monoclonal antibody is used.

### MEANS FOR SOLVING THE PROBLEMS

As a result of intensive study on the above-mentioned problems, the present inventors have discovered that, in order to detect adenovirus with higher sensitivity, it is effective to use a monoclonal antibody whose epitope is a specific amino acid sequence contained in adenovirus, thereby completing the present invention.

The invention is set out in the appended set of claims. That is, the present invention is as follows.
[1] A monoclonal antibody or an antigen-binding fragment thereof, which undergoes antigen-antibody reaction with a polypeptide having the sequence of the 546^{th} to 560^{th} amino acids in the amino acid sequence of SEQ ID NO: 1.
[2] An immunoassay for adenovirus, including performing the immunoassay of adenovirus by using antigen-antibody reaction between the monoclonal antibody or the antigen-binding fragment thereof according to [1] and adenovirus in a sample.
[3] The immunoassay according to [2], wherein the immunoassay is a sandwich method, and the monoclonal antibody or the antigen-binding fragment thereof is used as at least any one of a label or a solid phase.
[4] An immunoassay device for adenovirus, including the monoclonal antibody or the antigen-binding fragment thereof according to [1].

### EFFECTS OF THE INVENTION

The present invention can provide: a monoclonal antibody that makes it possible that adenovirus contained in a test specimen is detected and measured rapidly, simply, and with high-sensitivity; and an immunoassay for adenovirus and an immunoassay device therefor, for both of which the monoclonal antibody is used.

### BRIEF DESCRIPTION OF THE DRAWINGS

Fig. 1 is a diagram illustrating the results of Western blotting performed in Example 3.
Fig. 2 is a diagram illustrating the results obtained by staining, with CBB, the gel electrophoresed in Example 3.
Fig. 3-1 is a diagram illustrating the indexes 1 to 51 of the peptide library of the hexon protein of adenovirus type 3 GB strain.
Fig. 3-2 is a diagram illustrating the indexes 52 to 102 of the peptide library of the hexon protein of adenovirus type 3 GB strain.
Fig. 3-3 is a diagram illustrating the indexes 103 to 152 of the peptide library of the hexon protein of adenovirus type 3 GB strain.

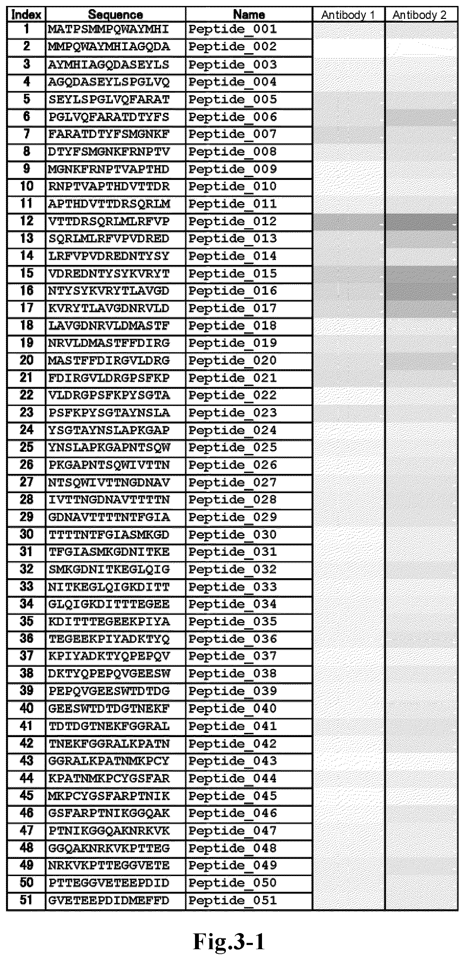

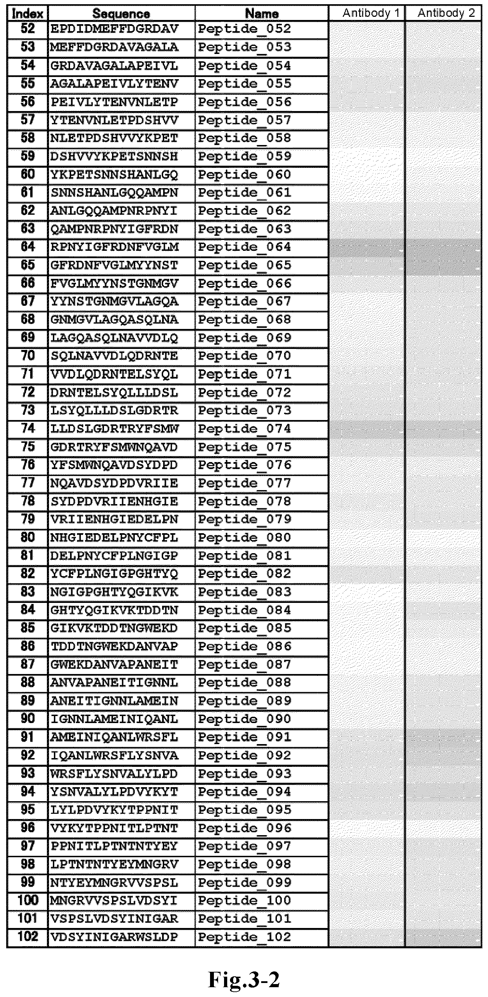

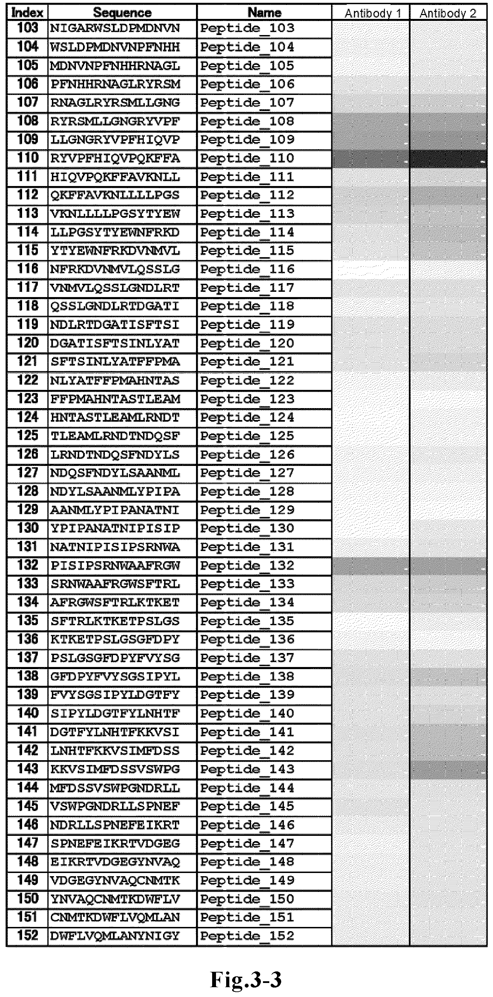

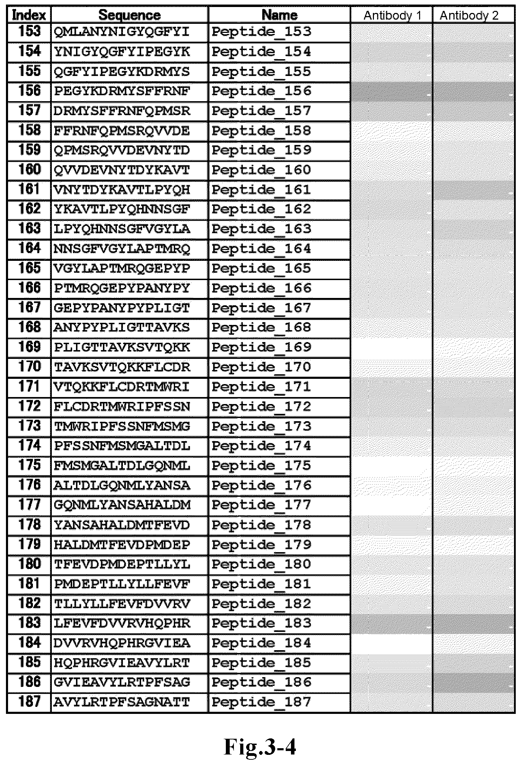

Fig. 3-4 is a diagram illustrating the indexes 153 to 187 of the peptide library of the hexon protein of adenovirus type 3 GB strain.

### MODE FOR CARRYING OUT THE INVENTION

Below, embodiments of the present invention will be described in detail.

### <Monoclonal Antibody or Antigen-binding Fragment thereof>

A monoclonal antibody or an antigen-binding fragment thereof according to the present invention undergoes antigen-antibody reaction with a polypeptide having the sequence of the 546^{th} to 560^{th} amino acids in the amino acid sequence of SEQ ID NO: 1. The amino acid sequence of SEQ ID NO: 1 is a sequence of the hexon monomer protein (GenBank Accession No. AB330084.1) of adenovirus type 3 GB strain, wherein the protein is constituted by 944 amino acid residues. Using a monoclonal antibody or an antigen-binding fragment thereof according to the present invention to detect adenovirus by Western blotting makes it possible that a specific signal due to antigen-antibody reaction is detected at a band considered as a monomer and corresponding to a molecular weight of approximately 100 kD and at a band considered as a trimer and corresponding to 200 to 300 kD. However, only a very weak reaction is recognized at a band considered as a monomer and corresponding to approximately 100 kD.

A monoclonal antibody or an antigen-binding fragment thereof disclosed herein undergoes antigen-antibody reaction with a polypeptide having at least one sequence selected from the group consisting of the region of the 21st to 131st amino acids, the region of the 266th to 412th amino acids, and the region of the 448th to 944th amino acids in the amino acid sequence of SEQ ID NO: 1. In this regard, the sequence of the 132nd to 265th amino acids and the sequence of the 413th to 447th amino acids in the amino acid sequence of SEQ ID NO: 1 are considered to be sequences having low conservability among the subtypes of adenovirus.

A monoclonal antibody or an antigen-binding fragment thereof disclosed herein undergoes antigen-antibody reaction with a polypeptide having at least one sequence selected from the group consisting of the region of the 21st to 115th amino acids, the region of the 266th to 385th amino acids, and the region of the 451st to 944th amino acids in the amino acid sequence of SEQ ID NO: 1.

A monoclonal antibody or an antigen-binding fragment thereof disclosed herein undergoes antigen-antibody reaction with a polypeptide having at least one sequence selected from the group consisting of the region of the 21st to 45th amino acids, the region of the 56th to 115th amino acids, the region of the 266th to 385th amino acids, the region of the 451st to 485th amino acids, the region of the 526th to 575th amino acids, the region of the 581st to 615th amino acids, the region of the 656th to 725th amino acids, the region of the 766th to 795th amino acids, the region of the 801st to 830th amino acids, the region of the 851st to 875th amino acids, and the region of the 886th to 944th amino acids in the amino acid sequence of SEQ ID NO: 1.

Using a monoclonal antibody or an antigen-binding fragment thereof that undergoes antigen-antibody reaction with a polypeptide having an amino acid sequence of any of these regions makes it possible to detect adenovirus with high sensitivity.

A monoclonal antibody or an antigen-binding fragment thereof according to the present invention has a basic structure composed of a heavy chain and a light chain, and the heavy chain and the light chain have the respective variable regions that can specifically bind to an antigen. V_{H} refers to the variable region of the heavy chain, and V_{L} refers to the variable region of the light chain. The variable region of the heavy chain and that of the light chain each contain amino acid sequences of complementarity-determining regions (CDR), that is, CDR1, CDR2, and CDR3, and a framework region (FR). For example, the variable region contains three or four FRs (for example, FR1, FR2, FR3, and optionally FR4) together with the three CDRs.

Examples of a monoclonal antibody of the present invention include quadruple-chain antibodies (for example, having two light chains and two heavy chains), recombinant antibodies, or modified antibodies (for example, chimeric antibodies, humanized antibodies, human antibodies, CDR transplanted antibodies, primatized antibodies, deimmunized antibodies, synhumanized (synhumanized) antibodies, half antibodies, and bispecific antibodies). The class of the monoclonal antibody is not limited to IgG, and may also be IgM or IgY.

In the present invention, an antigen-binding fragment of a monoclonal antibody is a fragment that is an antigen-binding site alone separated from the monoclonal antibody. Examples of such a fragment include fragments having specific antigen-binding capacity, such as Fab, Fab', F(ab')2, and single-chain antibodies (scFv) prepared by known methods.

### (Method of Preparing Monoclonal Antibody)

The monoclonal antibody of the present invention may be obtained by immunizing an animal with a complex or an extract containing adenovirus which contains the above-mentioned specific amino acid sequence and with which a monoclonal antibody of the present invention undergoes antigen-antibody reaction, or with the adenovirus or a partial peptide thereof by a known immunological method, and then preparing a hybridoma using cells of the immunized animal. The length of the peptide used for the immunization is not limited. Preferably, a peptide of not less than 5 amino acids, more preferably not less than 10 amino acids, may be used to provide the immunogen.

The immunogen can be obtained from a culture liquid, or can be obtained by incorporating, into a plasmid vector, DNA encoding an adenovirus antigen which contains the above-mentioned specific amino acid sequence and with which a monoclonal antibody of the present invention undergoes antigen-antibody reaction, and introducing the resulting vector to a host cell, followed by allowing expression of the adenovirus. Alternatively, an adenovirus antigen or the partial peptide thereof to be used as the immunogen can be expressed as a fusion protein with a protein exemplified below, and the expressed fusion protein can be used as the immunogen after purification or without purification. The preparation of the fusion protein can be carried out using, for example, Glutathion S-transferase (GST), maltose-binding protein (MBP), thioredoxin (TRX), Nus-tag, S-tag, HSV-tag, FRAG tag, polyhistidine tag, or the like which is commonly used as a "protein expression/purification tag" by those skilled in the art. Preferably, the fusion protein with such a protein is cleaved into the portion of the adenovirus antigen or the partial peptide thereof, and the tag portion, using a digestive enzyme, and subjected to separation/purification before use as the immunogen.

The preparation of the monoclonal antibody from the immunized animal can be easily carried out by the well-known method of Kohler *et al.* (Kohler et al., Nature, vol. 256, p. 495-497 (1975)). That is, antibody-producing cells such as spleen cells or lymphocytes are recovered from the immunized animal, and the recovered cells are fused with mouse myeloma cells by a conventional method to prepare hybridomas. The resulting hybridomas are cloned by the limiting dilution method or the like. Thereafter, a monoclonal antibody that undergoes antigen-antibody reaction with the antigen used for the immunization of the animal is selected from the monoclonal antibodies produced by the cloned hybridomas.

Purification of the monoclonal antibody from ascites or a culture supernatant may be carried out by a known immunoglobulin purification method. Examples of the method include fractionation by salting out using ammonium sulfate or sodium sulfate, PEG fractionation, ethanol fractionation, DEAE ion-exchange chromatography, and gel filtration. Depending on the species of the immunized animal and the class of the monoclonal antibody, affinity chromatography using a carrier to which any of protein A, protein G, and protein L is bound may be used for the purification.

### <Immunoassay>

In the present invention, using the above-mentioned monoclonal antibody or an antigen-binding fragment thereof makes it possible to detect adenovirus with very high sensitivity. In the following description preceding the Examples section, a "monoclonal antibody" means a "monoclonal antibody or an antigen-binding fragment thereof", unless otherwise obvious from the context.

In the present invention, adenovirus is detected by performing an immunoassay of adenovirus using antigen-antibody reaction between the above-mentioned monoclonal antibody and adenovirus in a sample. That a monoclonal antibody undergoes antigen-antibody reaction with adenovirus means that a monoclonal antibody reacts specifically with adenovirus. The term "specific" means that, in a liquid system containing a mixture of antigen proteins and the monoclonal antibody, the antibody does not cause antigen-antibody reaction with components other than the antigen proteins at a detectable level, or, even in cases where the antibody causes a certain binding reaction or association reaction with such a component, the reaction is evidently weaker than the antigen-antibody reaction between the antibody and the antigen proteins.

In the present invention, examples of immunoassays that can be used include any method well known to those skilled in the art, such as a competition method, condensation method, Western blotting, immunostaining method, sandwich method, and the like.

A sandwich method is preferable as an immunoassay in the present invention. The sandwich method per se is well known in the field of immunoassays, and can be carried out by, for example, immunochromatography or ELISA. These sandwich methods per se are well known, and the method of the present invention can be carried out in the same manner as a well-known sandwich method except that the above-mentioned specific monoclonal antibody is used.

In a sandwich method, two kinds of antibodies (an immobilized antibody immobilized in a solid phase and a labeled antibody) that recognize an antigen are used. In a method of the present invention, at least any one of these two kinds of antibodies is the above-mentioned monoclonal antibody of the present invention. In the case of the immobilized antibody immobilized in a solid phase, the amount of the antibody that can be immobilized per unit area is limited. To better achieve that object of the present invention which is to enhance the sensitivity, a monoclonal antibody according to the present invention is preferably used at least for the immobilized antibody. In cases where at least two antigens to be recognized by the monoclonal antibody are present in a single molecule or a single complex, the monoclonal antibody of a single kind can be used as a solid-phased antibody and a labelled antibody to perform a sandwich method.

In the immunoassay based on the detection principle of a sandwich method, any solid phase may be used as the solid phase on which the antibody is to be immobilized, as long as the antibody can be immobilized thereon by a known technique. The solid phase may be arbitrarily selected from known solid phases such as porous thin films (membranes) having a capillary action; particles, test tubes, and resin plates. Examples of the substance for labeling the antibody include enzymes, radioisotopes, fluorescent substances, luminescent substances, colored particles, and colloidal particles. Among the above-mentioned immunoassay methods using various materials, lateral-flow immunoassay methods using a membrane are preferred from the viewpoint of enabling simple and rapid clinical tests.

In cases where adenovirus is quantified or semi-quantified using the monoclonal antibody in the present invention, such quantification or semi-quantification involves "measurement" inevitably, and thus, is included in the "measurement" in the present invention. That is, in the present invention, "measurement" in an immunoassay includes any of quantification, semi-quantification, and detection.

### EXAMPLES

The present invention is described below by way of Examples. However, the present invention is not limited to the following Examples.

### (Example 1) Preparation of Anti-adenovirus Monoclonal Antibody

### 1. Preparation of Adenovirus Antigen

Adenovirus was allowed to infect mammalian cells sensitive thereto. The resulting cells were cultured for some days. Then, the culture liquid of the adenovirus-infected cells were inactivated by ultraviolet irradiation and made ready for use.

### 2. Preparation of Anti-adenovirus Monoclonal Antibody

BALB/c mice were immunized with the adenovirus-inactivated antigen prepared in 1, and kept for a certain period. From each mouse, the spleen was removed, and fusion with mouse myeloma cells (P3×63) was carried out by the method of Kohler *et al.* (Kohler et al., Nature, vol. 256, p. 495-497 (1975)) to obtain a plurality of hybridoma cell lines that produce anti-adenovirus antibodies.

The obtained cell line was intraperitoneally administered to pristane-treated BALB/c mice. About two weeks later, antibody-containing ascites was collected. From the ascites obtained, IgG was purified by affinity chromatography using a protein A column, to obtain a plurality of purified anti-adenovirus monoclonal antibodies.

In the below-mentioned Examples, two antibodies, an antibody 1 and an antibody 2, were used, which were selected from a plurality of the resulting anti-adenovirus monoclonal antibodies, considering reactivity and specificity.

### (Example 2) Immunoassay Device for Measuring Adenovirus

### 1. Immobilization of Anti-adenovirus Antibody on Nitrocellulose Membrane

The anti-adenovirus antibody (antibody 2) prepared in Example 1 was diluted with a buffer. The resulting liquid and an anti-mouse IgG antibody were made ready for use. The anti-adenovirus antibody and the anti-mouse IgG antibody were linearly applied respectively to the sample pad side and the absorber side of a nitrocellulose membrane lined with a PET film. Then, the nitrocellulose membrane was dried sufficiently under warm air to obtain a membrane having an anti-adenovirus antibody immobilized thereon.

### 2. Immobilization of Anti-adenovirus Antibody on Colored Polystyrene Particles

The anti-adenovirus antibody (antibody 1) prepared in Example 1 was covalently bound to colored polystyrene particles, and the colored polystyrene particles were suspended in a suspension. Then, colored polystyrene particles to which the anti-adenovirus antibodies were bound and which were dispersed sufficiently by ultrasonication were obtained. In the present description, the particles obtained here are referred to as "anti-adenovirus antibody-immobilized particles".

### 3. Application/Drying of Colored Polystyrene Particles to Which Anti-adenovirus Antibody is Bound

A predetermined amount of the anti-adenovirus antibody-immobilized particles prepared in 2 were applied to a glass-fiber non-woven fabric, and the non-woven fabric was then dried sufficiently under warm air. In the present description, the pad obtained here is referred to as a "labeled antibody pad".

### 4. Production of Adenovirus Test Device

The anti-adenovirus antibody-immobilized membrane prepared in 1 and the labelled antibody pads prepared in 2 and 3 were laminated with other members (backing sheet, absorption zone, and sample pad), and the resulting laminate was cut into a piece with a width of 5 mm, to provide an adenovirus test device.

### 5. Confirmation of Reactivity of Adenovirus Test Device

A culture liquid of adenovirus-infected cells of each type was diluted with a buffer to prepare a two-fold dilution series of each type of adenovirus.

The adenovirus diluted liquid prepared was added to a sample suspension (10 mM Tris (pH 8.0), 1 w/v% polyoxyethylene octylphenyl ether, 3 w/v% arginine, and 3 w/v% BSA), and 50 µL of the resulting mixture was added dropwise to the adenovirus test device produced in 4. Then, the test device was left to stand for 5 minutes.

In cases where coloring could be visually observed at both the position where the anti-mouse IgG antibody was applied and the position where the anti-adenovirus antibody was applied, the test result was evaluated as + (positive). In cases where coloring could be visually observed only at the position where the anti-mouse IgG antibody was applied and where coloring could not be visually observed at the position where the anti-adenovirus antibody was applied, the test result was evaluated as - (negative). In cases where coloring could not be visually observed at the position where the anti-mouse IgG antibody was applied, the test result was evaluated as invalid.

The lowest adenovirus concentration at which the test result was evaluated as positive was regarded as the lowest detection sensitivity. The results are shown in Table 1.

**[Table 1]**

| Adenovirus | | Lowest Detection Sensitivity | |
|---|---|---|---|
| Type | Species | TCID₅₀/Test | copies/Test |
| 1 | C | 1.95×10² | 2.50×10³ |
| 2 | C | 1.95×10² | 6.25×10³ |
| 3 | B | 2.79×10² | 1.25×10⁴ |
| 5 | C | 7.85×10¹ | 6.25×10³ |
| 6 | C | 5.58×10² | 3.13×10³ |
| 7 | B | 1.74×10² | 1.25×10⁴ |
| 8 | D | 1.95×10¹ | 1.25×10³ |
| 11 | B | 2.79×10² | 5.00×10⁴ |
| 19 | D | 2.44×10¹ | 1.25×10⁴ |
| 31 | A | 4.48×10² | 1.25×10⁵ |
| 37 | D | 3.14×10¹ | 2.50×10³ |

As shown in Table 1, it was possible to confirm that the immunoassay device used with the anti-adenovirus antibody of the present invention reacted with many types of adenovirus belonging to the species A to D.

In addition, it was possible to confirm that the device reacted with each of the subtypes: type 53, type 54, type 56, type 64, type 79, type 81, and type 85.

### 6. Confirmation of Specificity of Adenovirus Test Device

To the adenovirus test device produced in 4, 50 µL of a sample suspension containing virus that induces respiratory infection was added dropwise, and the test device was left to stand for 5 minutes.

In cases where coloring could be visually observed at both the position where the anti-mouse IgG antibody was applied and the position where the anti-adenovirus antibody was applied, the test result was evaluated as +. In cases where coloring could be visually observed only at the position where the anti-mouse IgG antibody was applied and where coloring could not be visually observed at the position where the anti-adenovirus antibody was applied, the test result was evaluated as -. In cases where coloring could not be visually observed at the position where the anti-mouse IgG antibody was applied, the test result was evaluated as invalid.

The results are shown in Table 2.

**[Table 2]**

| Virus | Measurement Results |
|---|---|
| Coxsackievirus type A9 | - |
| Coxsackievirus type B4 | - |
| Coxsackievirus type B5 | - |
| Coxsackievirus type B6 | - |
| Echo virus type 2 | - |
| Echo virus type 3 | - |
| Echo virus type 4 | - |
| Echo virus type 6 | - |
| Echo virus type 9 | - |
| Echo virus type 11 | - |
| Echo virus type 30 | - |
| Herpes simplex virus type 1 | - |
| Human Metapneumovirus type A | - |
| Human Metapneumovirus type B | - |
| Influenza virus A/New Caledonia/20/99 (H1N1) | - |
| Influenza virus A/Beijing/262/95 (H1N1) | - |
| Influenza virus A/New York/55/2004 (H3N2) | - |
| Influenza virus A/Hiroshima/52/2005 (H3N2) | - |
| Influenza virus B/Shanghai/361/2002 (Yamagata) | - |
| Influenza virus B/Malaysia/2506/2004 (Victoria) | - |
| Measles virus | - |
| Mumps virus | - |
| Parainfluenza virus type 1 | - |
| Parainfluenza virus type 2 | - |
| Parainfluenza virus type 3 | - |
| Parainfluenza virus type 4 | - |
| RS virus Long strain (type A) | - |
| RS virus CH-18 strain (type B) | - |

As shown in Table 2, it was possible to confirm that the adenovirus immunoassay device used with the anti-adenovirus antibody of the present invention reacted specifically with adenovirus, because the immunoassay device reacted with adenovirus, but exhibited no crossreactivity with the etiological virus of any other respiratory infection.

### 7. Performance Comparison of Adenovirus Test Device

The lowest detection sensitivity was compared between the adenovirus test device (the present device) prepared in 4 and a commercially available adenovirus kit.

Culture liquids of adenovirus-infected cells were each diluted with a buffer to prepare two-fold dilution series. To the present device, 50 µL of a sample suspension containing an adenovirus diluted liquid was added dropwise, and the present device was left to stand for 5 minutes and evaluated. The commercially available kit was used with a prescribed amount of adenovirus diluted liquid as a sample, and the test result was evaluated by performing a test in accordance with the document attached to each kit.

Assuming that the largest adenovirus dilution ratio at which the test result was evaluated as positive with the present device is "1", the largest dilution ratio at which the test result was evaluated as positive with a commercially available adenovirus kit was regarded as relative sensitivity, and is shown in Table 3.

**[Table 3]**

| Kit | Relative Sensitivity of Each Type (Species)* | | | | | | |
|---|---|---|---|---|---|---|---|
| | Type 1 (C) | Type 2 (C) | Type 3 (B) | Type 4 (E) | Type 11 (B) | Type 37 (D) | Type 54 (D) |
| Present Kit | 1 | 1 | 1 | 1 | 1 | 1 | 1 |
| Kit A | 1 | 1/2 | 1 | 1 | 1 | 1 | 1 |
| Kit B | 1/2 | 1/4 | 1/2 | 1/2 | 1/2 | 1/2 | 1/4 |
| Kit C | 1/8 | 1/8 | 1/8 | 1/20 | 1/20 | NT | NT |
| Kit D | 1 | 1/2 | 1/2 | 1/2 | 1/2 | 1/2 | 1/2 |
| Kit E | 1/16 | 1/32 | 1/100 | 1/80 | 1/400 | NT | NT |
| Kit F | 1/2 | 1/4 | 1/2 | 1/2 | 1/2 | 1/2 | 1/4 |
| Kit G | 1/4 | 1/8 | 1/8 | 1/8 | 1/4 | 1/4 | 1/4 |
| Kit H | 1/8 | 1/4 | 1/4 | 1/4 | 1/4 | 1/4 | 1/4 |

| | | | | | | | |
|---|---|---|---|---|---|---|---|
| NT: Not tested | | | | | | | |

As shown in Table 3, it was possible to confirm that the immunoassay device used with the anti-adenovirus antibody of the present invention had the highest reactivity with adenovirus type 2, and also had the highest reactivity with another type of adenovirus in the same manner as the kit A did.

### (Example 3) Antigen Recognition Site of Anti-adenovirus Monoclonal Antibody

The antigen recognition site of the anti-adenovirus monoclonal antibody obtained in Example 1 was verified by Western blotting and LC-MS/MS.

### 1. Preparation of Concentrated-Adenovirus Liquid

Adenovirus was allowed to infect A549 cells, and cultured. On culture day 7, the adenovirus-infected cells were recovered and disrupted by ultrasonication. Cell residues were removed from the disrupted-cell liquid by centrifugation to obtain a concentrated-adenovirus liquid.

### 2. Preparation of Sample without Reduction Treatment

A two-fold dilution series of the concentrated-adenovirus liquid obtained in 1 was prepared, and supplemented with reagents having the respective final concentrations: 62.5 mM Tris-HCI (pH 6.5), 10 w/v% glycerol, 2.3 w/v% SDS, and 0.05% BPB (dye). The resulting mixture was subjected to a conventional method SDS-PAGE without being thermally denatured.

### 3. Preparation of Sample with Reduction Treatment

A two-fold dilution series of the concentrated-adenovirus liquid obtained in 1 was prepared, and supplemented with reagents having the respective final concentrations: 62.5 mM Tris-HCI (pH 6.5), 10 w/v% glycerol, 2.3 w/v% SDS, 0.05% BPB (dye), and 5% 2-mercaptoethanol. After being thermally denatured at 95°C for 1 minute, the resulting mixture was subjected to a conventional method SDS-PAGE.

### 4. Western Blotting

The gels electrophoresed in 2 and 3 were transferred to a PVDF membrane. After blocking of the membrane using skim milk, the membrane was sufficiently washed with PBS-Tween. The membrane was then allowed to react with the anti-adenovirus antibody whose concentration was adjusted to 3.8 µg/mL using PBS-Tween, at room temperature for 1 hour. After sufficiently washing the membrane with PBS-Tween, the membrane was allowed to react with a 3000-fold diluted HRP-labeled anti-mouse antibody at room temperature for 1 hour. After sufficiently washing the membrane with PBS-Tween, signals were detected using a chemiluminescence detection reagent.

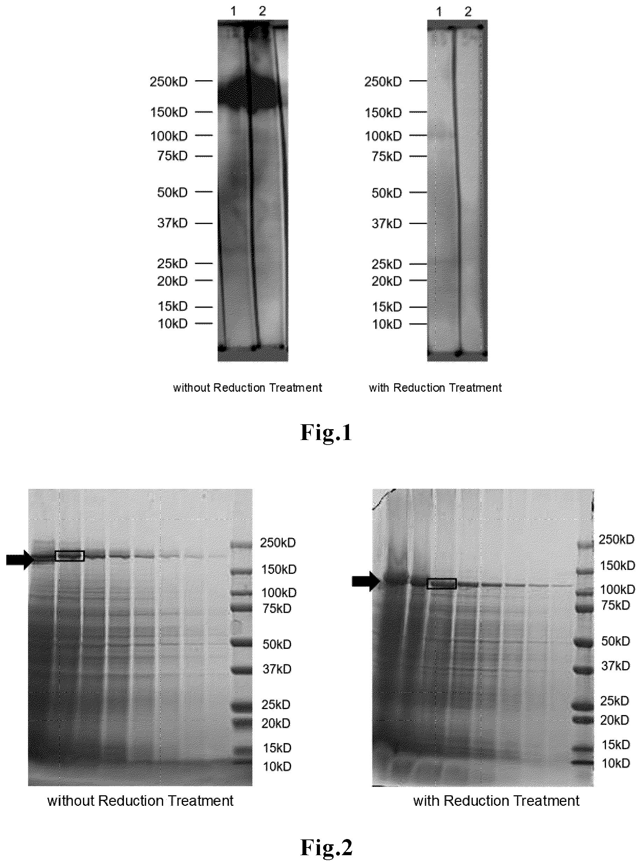

The results of Western Blotting are shown in Fig. 1.

A shown in Fig. 1, two antibodies (the antibody 1 and the antibody 2) produced in Example 1 reacted strongly with the approximately 200 kD protein (hexon trimer) contained in the sample obtained in 2 (without reduction treatment) (the left diagram). It is considered that the reduction treatment caused the hexon trimer to become a monomer, and it was recognized that the sample obtained in 3 (with reduction treatment) reacted very weakly with the approximately 100 kD protein (hexon monomer) (right diagram).

The results obtained by staining, with CBB, the gels electrophoresed in 2 and 3 are shown in Fig. 2.

As shown by the arrow in Fig. 2, the main protein contained in the sample obtained in 2 (without reduction treatment) was found at approximately 200 kD (the left diagram), and that contained in the sample obtained in 3 (with reduction treatment) was found at 100 to 150 kD (the right diagram). Each stained region denoted by a rectangle in the diagram was cut out, then hydrolyzed with trypsin, and analyzed by LC-MS/MS to give an amino acid sequence. The peptide fragment obtained was analyzed using Mascot (Ver. 2.5) (from Matrix Science Inc.) and Scaffold (from Proteome Software, Inc.), resulting in revealing that both of the stained regions contained a hexon protein of adenovirus as a main constituent.

Fig. 1 and Fig. 2 have revealed that both of the two antibodies obtained in Example 1 reacted more strongly with a protein of a hexon trimer than with a protein of a hexon monomer, and reacted weakly with a monomeric hexon protein. It has been demonstrated that the reaction between the antibody according to the present invention and a hexon monomer is weak, but that the antibody according to the present invention, which undergoes antigen-antibody reaction with a specific sequence of the monomer, is very effective for detection of adenovirus.

### (Example 4) Reactivity Analysis of Anti-adenovirus Monoclonal Antibody to Adenovirus Hexon Protein by Peptide Microarray PepStar (Manufactured by JPT Peptide Technologies Inc., hereinafter referred to as "Pepstar").

### 1. Production of PepStar

On the basis of the information on the amino acid sequence of the hexon protein (GenBank Accession No. AB330084.1) of adenovirus type 3 GB strain, peptide microarrays Pepstars (manufactured by JPT Peptide Technologies GmbH) were purchased, in which the peptides listed in the peptide library shown in Table 4 were immobilized.

**[Table 4-1]**

| Table 4 Peptide Library of Hexon Protein (GenBank Accession No. AB330084.1) of Adenovirus Type 3 GB Strain | | |
|---|---|---|
| **Index** | **Sequence** | **Name** |
| **1** | MATPSMMPQWAYMHI | Peptide_001 |
| **2** | MMPQWAYMHIAGQDA | Peptide_002 |
| **3** | AYMHTIAGQDASEYLS | Peptide_003 |
| **4** | AGQDASEYLSPGLVQ | Peptide_004 |
| **5** | SEYLSPGLVQFARAT | Peptide_005 |
| **6** | PGLVQFARATDTYFS | Peptide_006 |
| **7** | FARATDTYFSMGNKF | Peptide_007 |
| **8** | DTYFSMGNKFRNPTV | Peptide_008 |
| **9** | MGNKFRNPTVAPTHD | Peptide_009 |
| **10** | RNPTVAPTHDVTTDR | Peptide_010 |
| **11** | APTHDVTTDRSQRLM | Peptide_011 |
| **12** | VTTDRSQRLMLRFVP | Peptide_012 |
| **13** | SQRLMLRFVPVDRED | Peptide_013 |
| **14** | LRFVPVDREDNTYSY | Peptide_014 |
| **15** | VDREDNTYSYKVRYT | Peptide_015 |
| **16** | NTYSYKVRYTLAVGD | Peptide_016 |
| **17** | KVRYTLAVGDNRVLD | Peptide _017 |
| **18** | LAVGDNRVLDMASTF | Peptide_018 |
| **19** | NRVLDMASTFFDIRG | Peptide_019 |
| **20** | MASTFFDIRGVLDRG | Peptide_020 |
| **21** | FDIRGVLDRGPSFKP | Peptide_021 |
| **22** | VLDRGPSFKPYSGTA | Peptide_022 |
| **23** | PSFKPYSGTAYNSLA | Peptide_023 |
| **24** | YSGTAYNSLAPKGAP | Peptide_024 |
| **25** | YNSLAPKGAPNTSQW | Peptide_025 |
| **26** | PKGAPNTSQWIVTTN | Peptide_026 |
| **27** | NTSQWIVTTNGDNAV | Peptide_027 |
| **28** | IVTTNGDNAVTTTTN | Peptide_028 |
| **29** | GDNAVTTTTNTFGIA | Peptide_029 |
| **30** | TTTTNTFGIASMKGD | Peptide_030 |
| **31** | TFGIASMKGDNITKE | Peptide_031 |
| **32** | SMKGDNITKEGLQIG | Peptide_032 |
| **33** | NITKEGLQIGKDITT | Peptide_033 |
| **34** | GLQIGKDITTTEGEE | Peptide_034 |
| **35** | KDITTTEGEEKPIYA | Peptide_035 |
| **36** | TEGEEKPIYADKTYQ | Peptide_036 |
| **37** | KPIYADKTYQPEPQV | Peptide_037 |
| **38** | DKTYQPEPQVGEESW | Peptide_038 |
| **39** | PEPQVGEESWTDTDG | Peptide_039 |
| **40** | GEESWTDTDGTNEKF | Peptide_040 |
| **41** | TDTDGTNEKFGGRAL | Peptide_041 |
| **42** | TNEKFGGRALKPATN | Peptide_042 |
| **43** | GGRALKPATNMKPCY | Peptide_043 |
| **44** | KRATNMKPCYGSRAR | Peptide_044 |
| **45** | MKPCYGSFARPTNIK | Peptide_045 |
| **46** | GSFARPTNIKGGQAK | Peptide_046 |
| **47** | PTNIKGGQAKNRKVK | Peptide_047 |
| **48** | GGQAKNRKVKPTTEG | Peptide_048 |
| **49** | NRKVKPTTEGGVETE | Peptide_049 |
| **50** | PTTEGGVETEEPDID | Peptide_050 |
| **51** | GVETEEPDIDMEFFD | Peptide_051 |

**[Table 4-2]**

| **Index** | **Sequence** | **Name** |
|---|---|---|
| **52** | EPDIDMEFFDGRDAV | Peptide_052 |
| **53** | MEFFDGRDAVAGATA | Peptide_053 |
| **54** | GRDAVAGALAPEIVL | Peptide_054 |
| **55** | AGALAPEIVLYTENV | Peptide 055 |
| **56** | PEIVLYTENVNLETP | Peptide_056 |
| **57** | YTENVNLETPDSHVV | Peptide_057 |
| **58** | NLETPDSHVVYKPET | Peptide_058 |
| **59** | DSHVVYKPETSNNSH | Peptide_059 |
| **60** | YKPETSNNSHANLGQ | Peptide_060 |
| **61** | SNNSHANLGQQAMPN | Peptide_061 |
| **62** | ANLGQQAMPNRPNYI | Peptide_062 |
| **63** | QAMPNRPNYIGFRDN | Peptide_063 |
| **64** | RPNYIGFRDNFVGLM | Peptide_064 |
| **65** | GFRDNFVGIMYYNST | Peptide_065 |
| **66** | FVGLMYYNSTGNMGV | Peptide_066 |
| **67** | YYNSTGNMGVLAGQA | Peptide_067 |
| **68** | GNMGVLAGQASQLNA | Peptide_068 |
| **69** | LAGQASQLNAVVDLQ | Peptide_069 |
| **70** | SQLNAVVDLQDRNTE | Peptide_070 |
| **71** | VVDLQDRNTELSYQL | Peptide_071 |
| **72** | DRNTELSYQLLLDSL | Peptide_072 |
| **73** | LSYQLLLDSLGDRTR | Peptide_073 |
| **74** | LLDSLGDRTRYFSMN | Peptide_074 |
| **75** | GDRTRYFSMWNQAVD | Peptide_075 |
| **76** | YFSMWNQAVDSYDPD | Peptide_076 |
| **77** | NQAVDSYDPDVRIIE | Peptide_077 |
| **78** | SYDPDVRIIENHGIE | Peptide_078 |
| **79** | VRIIENHGIEDELPN | Peptide_079 |
| **80** | NHGIEDELPNYCFPL | Peptide_080 |
| **81** | DELPNYCFPLNGIGP | Peptide_081 |
| **82** | YCFPLNGIGPGHTYQ | Peptide_082 |
| **83** | NGIGPGHTYQGIKVK | Peptide_083 |
| **84** | GHTYQGIKVKTDDTN | Peptide_084 |
| **85** | GIKVKTDDTNGWEKD | Peptide_085 |
| **86** | TDDTNGWEKDANVAP | Peptide_086 |
| **87** | GWEKDANVAPANEIT | Peptide_087 |
| **88** | ANVAPANEITIGNNL | Peptide_088 |
| **89** | ANEITIGNNLAMEIN | Peptide_089 |
| **90** | IGNNLAMEINIQANL | Peptide_090 |
| **91** | AMEINIQANLWRSFL | Peptide_091 |
| **92** | IQANLWRSFLYSNVA | Peptide_092 |
| **93** | WRSFLYSNVALYLPD | Peptide_093 |
| **94** | YSNVALYLPDVYKYT | Peptide_094 |
| **95** | LYLPDVYKYTPPNIT | Peptide_095 |
| **96** | VYKYTPPNITLPTNT | Peptide_096 |
| **97** | PPNITLPTNTNTYEY | Peptide_097 |
| **98** | LPTNTNTYEYMNGRV | Peptide_098 |
| **99** | NTYEYMNGRVVSPSL | Peptide_099 |
| **100** | MNGRVVSPSLVDSYI | Peptide**_**100 |
| **101** | VSPSLVDSYINIGAR | Peptide_101 |
| **102** | VDSYINIGARWSLDP | Peptide_102 |

**[Table 4-3]**

| **Index** | **Sequence** | **Name** |
|---|---|---|
| **103** | NIGARWSLDPMDNVN | Peptide_103 |
| **104** | WSLDPMDNVNPFNHH | Peptide_104 |
| **105** | MDNVNPFNHHRNAGL | Peptide_105 |
| **106** | PFNHHRNAGLRYRSM | Peptide_106 |
| **107** | RNAGLRYRSMLLGNG | Peptide_107 |
| **108** | RYRSMLLGNGRYVPF | Peptide_108 |
| **109** | LLGNGRYVPFHIQVP | Peptide_109 |
| **110** | RYVPFHIQVPQKFFA | Peptide_110 |
| **111** | HIQVPQKFFAVKNLL | Peptide_111 |
| **112** | QKFFAVKNLLLLPGS | Peptide_112 |
| **113** | VKNLLLLPGSYTYEW | Peptide_113 |
| **114** | LLPGSYTYEWNFRKD | Peptide_114 |
| **115** | YTYEWNFRKDVNMVL | Peptide_115 |
| **116** | NFREKDVNMVLQSSLG | Peptide_116 |
| **117** | VNMVLQSSLGNDLRT | Peptide_117 |
| **118** | QSSLGNDLRTDGATI | Peptide_118 |
| **119** | NDLRTDGATISFTSI | Peptide_119 |
| **120** | DGATISFTSINLYAT | Peptide_120 |
| **121** | SFTSINLYATFFPMA | Peptide_121 |
| **122** | NLYATFFPMAHNTAS | Peptide_122 |
| **123** | FFPMAHNTASTLEAM | Peptide_123 |
| **124** | HNTASTLEAMLRNDT | Peptide_124 |
| **125** | TLEAMLRNDTNDQSF | Peptide_125 |
| **126** | LRNDTNDQSFNDYLS | Peptide_126 |
| **127** | NDQSFNDYLSAANML | Peptide_127 |
| **128** | NDYLSAANMLYPIPA | Peptide_128 |
| **129** | AANMLYPIPANATNI | Peptide_129 |
| **130** | YPIPANATNIPISIP | Peptide_130 |
| **131** | NATNIPISIPSRNWA | Peptide_131 |
| **132** | PISIPSRNWAAFRGW | Peptide_132 |
| **133** | SRNWAAFRGWSFTRL | Peptide_133 |
| **134** | AFRGWSFTRLKTKET | Peptide_134 |
| **135** | SFTRLKTKETPSLGS | Peptide_135 |
| **136** | KTKETPSLGSGFDPY | Peptide_136 |
| **137** | PSLGSGFDPYFVYSG | Peptide_137 |
| **138** | GFDPYFVYSGSIPYL | Peptide_138 |
| **139** | FVYSGSIPYLDGTFY | Peptide_139 |
| **140** | SIPYLDGTFYLNHTF | Peptide_140 |
| **141** | DGTFYLNHTFKKVSI | Peptide_141 |
| **142** | LNHTFKKVSIMFDSS | Peptide_142 |
| **143** | KKVSIMFDSSVSWPG | Peptide_143 |
| **144** | MFDSSVSWPGNDRLL | Peptide_144 |
| **145** | VSWPGNDRLLSPNEF | Peptide_145 |
| **146** | NDRLLSPNEFEIKRT | Peptide_146 |
| **147** | SPNEFEIKRTVDGEG | Peptide_147 |
| **148** | EIKRTVDGEGYNVAQ | Peptide_148 |
| **149** | VDGEGYNVAQCNMTK | Peptide_149 |
| **150** | YNVAQCNMTKDWFLYV | Peptide_150 |
| **151** | CNMTKDWFLVQMLAN | Peptide_151 |
| **152** | DWFLVQMLANYNIGY | Peptide_152 |

**[Table 4-4]**

| **Index** | **Sequence** | **Name** |
|---|---|---|
| **153** | QMLANYNIGYQGFYI | Peptide_153 |
| **154** | YNIGYQGFYIPEGYK | Peptide_154 |
| **155** | QGFYIPEGYKDRMYS | Peptide_155 |
| **156** | PEGYKDRMYSFFRNF | Peptide_156 |
| **157** | DRMYSFFRNFQPMSR | Peptide_157 |
| **158** | FFRNFQPMSRQVVDE | Peptide_158 |
| **159** | QPMSRQVVDEVNYTD | Peptide_159 |
| **160** | QVVDEVNYTDYKAVT | Peptide_160 |
| **161** | VNYTDYKAVTLPYQH | Peptide_161 |
| **162** | YKAVTLPYQHNNSGF | Peptide_162 |
| **163** | LPYQHNNSGFVGYLA | Peptide_163 |
| **164** | NNSGFVGYLAPTMRQ | Peptide_164 |
| **165** | VGYLAPTMRQGEPYP | Peptide_165 |
| **166** | PTMRQGEPYPANYPY | Peptide_166 |
| **167** | GEPYPANYPYPLIGT | Peptide_167 |
| **168** | ANYPYPLIGTTAVKS | Peptide_168 |
| **169** | PLIGTTAVKSVTQKK | Peptide_169 |
| **170** | TAVKSVTQKKFLCDR | Peptide_170 |
| **171** | VTQKKFLCDRTMWRI | Peptide_171 |
| **172** | FLCDRTMWRIPFSSN | Peptide_172 |
| **173** | TMWRIPFSSNFMSMG | Peptide_173 |
| **174** | PFSSNEMSMGALTDL | Peptide_174 |
| **175** | FMSMGALTDLGQNML | Peptide_175 |
| **176** | ALTDLGQNMLYANSA | Peptide_176 |
| **177** | GQNMLYANSAHALDM | Peptide_177 |
| **178** | YANSAHALDMTFEVD | Peptide_178 |
| **179** | HALDMTFEVDPMDEP | Peptide_179 |
| **180** | TFEVDPMDEPTLLYL | Peptide_180 |
| **181** | PMDEPTLLYLLFEVF | Peptide_181 |
| **182** | TLLYLLFEVFDVVRV | Peptide_182 |
| **183** | LFEVFDVVRVHQPHR | Peptide_183 |
| **184** | DVVRVHQPHRGVIEA | Peptide_184 |
| **185** | HQPHRGVIEAVYLRT | Peptide_185 |
| **186** | GVIEAVYLRTPFSAG | Peptide_186 |
| **187** | AVYLRTPFSAGNATT | Peptide_187 |

### 2. Reactivity Analysis of Anti-adenovirus Monoclonal Antibody by Pepstar

The two kinds of anti-adenovirus antibodies produced in Example 1 were diluted with a buffer, and the antibodies were each allowed to react on Pepstar at 30°C for 1 hour. After the reaction, 1 µg/ml secondary fluorescent-labeled anti-mouse IgG antibody was added to the corresponding well, and allowed to react for 1 hour. The reaction product in the well was washed and dried, and then, the slide was scanned with a 635 nm high-resolution laser scanner to obtain a fluorescence intensity profile. The image acquired was quantified to obtain the average pixel value of each peptide.

The result obtained was visualized to prepare a heat map (Fig. 3) that depicted the fluorescence intensity in accordance with colors from white (no binding) to red (strong binding) to compare the individual binding regions.

The result of the heat map in Fig. 3 clarified the region of the reaction of the two kinds of anti-adenovirus monoclonal antibodies prepared in Example 1 to the hexon protein of adenovirus.

## Claims

1. A monoclonal antibody or an antigen-binding fragment thereof, which undergoes antigen-antibody reaction with a polypeptide consisting of the sequence of the 546th to 560th amino acids in the amino acid sequence of SEQ ID NO: 1.

2. A method for detecting an adenovirus, the method comprising performing the immunoassay of adenovirus by using antigen-antibody reaction between the monoclonal antibody or the antigen-binding fragment thereof according to claim 1 and adenovirus in a sample.

3. The method according to claim 2, wherein the immunoassay is a sandwich method, and the monoclonal antibody or the antigen-binding fragment thereof is used as at least any one of a label or a solid phase.

4. An immunoassay device for adenovirus, comprising a sample pad and/or a polystyrene particle upon which the monoclonal antibody or the antigen-binding fragment thereof according to claim 1 is immobilized.

## Patentansprüche

1. Monoklonaler Antikörper oder Antigen-bindendes Fragment davon, der/das eine Antigen-Antikörper-Reaktion mit einem Polypeptid eingeht, das aus der Sequenz der 546. bis 560. Aminosäuren in der Aminosäuresequenz von SEQ ID NO: 1 besteht.

2. Verfahren zum Detektieren eines Adenovirus, wobei das Verfahren das Durchführen des Immunassays für Adenoviren unter Verwendung der Antigen-Antikörper-Reaktion zwischen einem monoklonalen Antikörper oder einem Antigen-bindenden Fragment davon nach Anspruch 1 und dem Adenovirus in einer Probe umfasst.

3. Verfahren nach Anspruch 2, wobei das Immunassay ein Sandwich-Verfahren ist und der monoklonale Antikörper oder das Antigen-bindende Fragment davon als zumindest eines aus einer Markierung oder einer festen Phase verwendet wird.

4. Immunassay-Vorrichtung für Adenoviren, umfassend ein Probenkissen und/oder einen Polystyrolpartikel, auf dem ein monoklonaler Antikörper oder ein Antigen-bindendes Fragment davon nach Anspruch 1 immobilisiert ist.

## Revendications

1. Anticorps monoclonal ou fragment de liaison à un antigène de celui-ci, qui subit une réaction antigène-anticorps avec un polypeptide constitué de la séquence des 546ème à 560ème acides aminés dans la séquence d'acides aminés de SEQ ID NO : 1.

2. Procédé de détection d'un adénovirus, le procédé comprenant la réalisation du dosage immunologique d'adénovirus en utilisant une réaction antigène-anticorps entre l'anticorps monoclonal ou le fragment de liaison à un antigène de celui-ci selon la revendication 1 et l'adénovirus dans un échantillon.

3. Procédé selon la revendication 2, dans lequel le dosage immunologique est un procédé sandwich, et l'anticorps monoclonal ou le fragment de liaison à un antigène de celui-ci est utilisé comme au moins l'un quelconque d'un marqueur ou d'une phase solide.

4. Dispositif de dosage immunologique d'adénovirus, comprenant un plot d'échantillon et/ou une particule de polystyrène sur lequel ou laquelle l'anticorps monoclonal ou le fragment de liaison à un antigène de celui-ci selon la revendication 1 est immobilisé.
